# EUROPEAN PATENT APPLICATION

(11) **EP 2 534 963 A1**
(43) Date of publication of application: **19.12.2012**
(21) Application number: 11742291.5
(22) Date of filing: 10.02.2011
(51) Int. Cl.: A23L 1/305, A23K 1/16, C07K 1/12, C07K 4/12, C12P 21/06

(54) **PROTEIN SYNTHESIS PROMOTER**

(30) Priority: 12.02.2010 JP 2010029306
(71) Applicant: Megmilk Snow Brand Co., Ltd., Sapporo-shi Hokkaido 0650043 (JP)
(72) Inventor: MIURA, Susumu, Sapporo-shi Hokkaido 065-0043 (JP)
(74) Representative: Wakerley, Helen Rachael
(86) International application number: PCT/JP2011/052841
(87) International publication number: WO 2011/099548

(57) **Abstract**

A protein synthesis promoter that exhibits a protein synthesis-promoting effect includes a whey protein hydrolyzate having a molecular weight distribution that is within a range of 10 kDa or less and has a main peak of 200 Da to 3 kDa, an average peptide length (APL) of 2 to 8, a free amino acid content of 20% or less, a branched-chain amino acid content of 20% or more, and an antigenicity equal to or less than 1/100,000th of that of β-lactoglobulin.

## Description

### TECHNICAL FIELD

The invention relates to a protein synthesis promoter that includes a whey protein hydrolyzate that exhibits low bitterness, stability, and safety as an active ingredient.
The invention also relates to a protein synthesis-promoting food, a protein synthesis-promoting drink, a protein synthesis-promoting nutrient composition, and a protein synthesis-promoting feed that include a whey protein hydrolyzate that exhibits low bitterness, stability, and safety as an active ingredient.

### BACKGROUND ART

A protein biosynthesis process includes an mRNA transcription process that occurs due to an RNA polymerase based on a DNA base sequence, and a translation process in which amino acids are polymerized in the ribosomes based on the mRNA base sequence information to form a polypeptide chain. The protein biosynthesis process is controlled by various regulators. In particular, S6 kinase 1 (S6K1) and 4E-BP1 are used as indices of the translation process.

It is known that branched-chain amino acid (BCAA) (particularly leucine) independently promotes protein synthesis, and may be used to build muscle. BCAA promotes protein synthesis by activating the protein translation process. However, since BCAA is produced by individually preparing valine, leucine, and isoleucine, and mixing them, BCAA is expensive for regular use.

Cow milk or a dairy product is often considered to be the cause of a food allergy. In particular, a whey protein that is not contained in human breast milk is considered to function as an allergen. Therefore, a method that decreases the allergenicity of a whey protein by hydrolyzing the whey protein using a protease has been proposed (see Patent Document 1, for example).
It has been confirmed that a whey protein hydrolyzate produced by the method disclosed in Patent Document 2 has an antigenicity equal to or less than 1/10,000th of that of β-lactoglobulin and a whey protein using inhibition ELISA (see Non-Patent Document 1).
It has been found that a whey protein hydrolyzate has a fat accumulation-inhibiting effect via oral administration. Therefore, a whey protein hydrolyzate has attracted attention as a material that has low allergenicity and high functionality.

### RELATED-ART DOCUMENT

### PATENT DOCUMENT

Patent Document 1: JP-A-2-138991
Patent Document 2: WO2008/111562

### NON-PATENT DOCUMENT

Non-patent Document 1: Japanese Journal of Pediatric Allergy and Clinical Immunology, 1, 36 (1987)

### SUMMARY OF THE INVENTION

### TECHNICAL PROBLEM

An object of the invention is to provide a protein synthesis promoter that is cheaper than BCAA, and has a protein synthesis-promoting effect.

### SOLUTION TO PROBLEM

The inventor of the invention conducted extensive studies, and found that a whey protein hydrolyzate obtained by hydrolyzing a whey protein contained in cow milk has a protein synthesis-promoting effect. This finding has led to the completion of the invention.
Specifically, the invention provides the following protein synthesis promoter, as well as the following protein synthesis-promoting food, protein synthesis-promoting drink, and protein synthesis-promoting feed that include the protein synthesis promoter.

(1) A protein synthesis promoter including a whey protein hydrolyzate as an active ingredient, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.
(2) The protein synthesis promoter according to (1), wherein the whey protein hydrolyzate is obtained by performing a hydrolysis step that hydrolyzes and thermally denatures a whey protein at a pH of 6 to 10 and a temperature of 50 to 70°C using a heat-resistant protease, and an inactivation step that inactivates the protease by heating.
(3) The protein synthesis promoter according to (1), wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, and an inactivation step that inactivates the protease by heating.
(4) The protein synthesis promoter according to (1), wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, an inactivation step that inactivates the protease by heating, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da.
(5) A protein synthesis-promoting food, a protein synthesis-promoting drink, a protein synthesis-promoting nutrient composition, or a protein synthesis-promoting feed including the protein synthesis promoter according to any one of (1) to (4).
(6) A method of producing a protein synthesis promoter including a hydrolysis step that hydrolyzes and thermally denatures a whey protein at a pH of 6 to 10 and a temperature of 50 to 70°C using a heat-resistant protease, and an inactivation step that inactivates the protease by heating.
(7) The method according to (6), further including, before the hydrolysis step, a preliminary hydrolysis step that hydrolyzes the whey protein at a pH of 6 to 10 and a temperature of 20 to 55°C using a protease.
(8) The method according to (6) or (7), further including, after the inactivation step, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da.
(9) A protein synthesis-promoting method including administering a whey protein hydrolyzate in an amount of 10 g/day or more, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.

### ADVANTAGEOUS EFFECTS OF INVENTION

The protein synthesis promoter of the present invention exhibits a remarkable protein synthesis-promoting effect. The protein synthesis promoter promotes protein biosynthesis by activating the initiation of a translation process that synthesizes polypeptides based on mRNA information.

### DESCRIPTION OF EMBODIMENTS

The invention is described in detail below.

### Active ingredient

A whey protein hydrolyzate that is used as an active ingredient of a protein synthesis promoter according to one embodiment of the invention has a molecular weight distribution that is within a range of 10 kDa or less and has a main peak of 200 Da to 3 kDa, an average peptide length (APL) of 2 to 8, a free amino acid content of 20% or less, a branched-chain amino acid content of 20% or more, and an antigenicity equal to or less than 1/100,000th of that of β-lactoglobulin.
Since the whey protein hydrolyzate has an antigenicity equal to or less than 1/100,000th of that of β-lactoglobulin and a whey protein, the whey protein hydrolyzate is highly safe in terms of a food allergy.
An aqueous solution of the whey protein hydrolyzate is transparent, and has a bitterness of about 2. Therefore, the whey protein hydrolyzate does not impose limitations to the protein synthesis promoter in terms of flavor (taste) and appearance. In particular, a large amount of the whey protein hydrolyzate can be added to the protein synthesis promoter even when transparency is particularly desired. The water-solubility of the whey protein hydrolyzate can be improved by filtering the whey protein hydrolyzate using an ultrafiltration (UF) membrane or a microfiltration (MF) membrane.
A protein synthesis-promoting food, a protein synthesis-promoting drink, a protein synthesis-promoting nutrient composition, and a protein synthesis-promoting feed that exhibit a protein synthesis-promoting effect, and are safe can be provided by utilizing the protein synthesis promoter as an active ingredient.
Since the protein synthesis promoter according to one embodiment of the invention is produced using a whey protein as a raw material, the protein synthesis promoter can be produced easily and economically.

### Production method

The whey protein hydrolyzate included in the protein synthesis promoter according to one embodiment of the invention is obtained by hydrolyzing and thermally denaturing a whey protein at a pH of 6 to 10 and a temperature of 50 to 70°C using a heat-resistant protease, and inactivating the protease by heating. The yield of the whey protein hydrolyzate can be improved by preliminarily hydrolyzing the whey protein at a pH of 6 to 10 and a temperature of 20 to 55°C using a protease, and then immediately hydrolyzing the whey protein under the above conditions without cooling the whey protein.
The protein synthesis-promoting effect can be further improved by concentrating the whey protein hydrolyzate prepared as described above using an ultrafiltration (UF) membrane having a molecular weight cut-off of 1 to 20 kDa (preferably 2 to 10 kDa) and/or a microfiltration (MF) membrane having a molecular weight cut-off of 100 to 500 Da (preferably 150 to 300 Da). The whey protein hydrolyzate thus obtained exhibits lower bitterness and improved transparency.
The term "whey protein" used herein refers to whey of a mammal (e.g., cow, buffalo, goat, or human), an aggregate, a powder, or a purified product thereof. The whey protein is used in a state of an aqueous solution when hydrolyzing the whey protein using a protease.
The pH of the whey protein aqueous solution is adjusted to 6 to 10. Note that the whey protein aqueous solution normally has a pH within the above range. When it is necessary to adjust the pH of the whey protein aqueous solution, the pH of the whey protein aqueous solution is adjusted to 6 to 10 using a solution of an acid (e.g., hydrochloric acid, citric acid, or lactic acid) or an alkali (e.g., caustic soda, calcium hydroxide, or sodium phosphate). The whey protein aqueous solution is heated to 50 to 70°C. It is preferable to add the heat-resistant protease before heating the whey protein aqueous solution so that hydrolysis also occurs during heating (i.e., the yield is improved).
The optimum temperature for a normal protease is 40°C or less, and the optimum temperature for a heat-resistant protease is 45°C or more. An arbitrary heat-resistant protease may be used as long as the heat-resistant protease has an optimum temperature of 45°C or more. Examples of such a heat-resistant protease include papain, Protease S (trade name), Proleather (trade name), Thermoase (trade name), Alcalase (trade name), Protin A (trade name), and the like. It is preferable to use a heat-resistant protease that has a residual activity of 10% or more when heated at 80°C for 30 minutes. It is more effective to use a plurality of proteases in combination. The reaction time is preferably about 30 minutes to about 10 hours.
The reaction solution is then heated to inactivate the protease. The protease may be inactivated by heating the reaction solution at 100°C or more for 10 seconds or more.
After inactivating the protease, the reaction solution is centrifuged. The supernatant liquid is then collected, and dried to obtain a powdery product. Since a precipitate that occurs due to centrifugation is at a lower level of hypoallergenic property as compared with the supernatant liquid, it is preferable to remove the precipitate. Note that the reaction solution may be dried and used directly as long as there is not a problem of antigenicity.
It was confirmed that the whey protein hydrolyzate obtained by the above method has an antigenicity equal to or less than 1/100,000th of that of β-lactoglobulin and a whey protein when measured by inhibition ELISA. Therefore, the whey protein hydrolyzate is highly safe. An aqueous solution of the whey protein hydrolyzate is transparent, and has a bitterness of about 2. Therefore, the whey protein hydrolyzate rarely poses a problem in terms of flavor (taste) and appearance. Note that the transparency and the bitterness of the whey protein hydrolyzate were evaluated by the following methods.
Transparency evaluation method: A 1% whey protein hydrolyzate solution was prepared, and the absorbance at 650 nm was measured.
Bitterness evaluation method: A 10% whey protein hydrolyzate solution was prepared, and the bitterness of the solution was evaluated using quinine hydrochloride (bitter substance). A whey protein hydrolyzate having a bitterness of 2 or less (see Table 1) can be used for food, drink, or the like.
The APL of the whey protein hydrolyzate may be determined by HPLC or the like.

**TABLE 1**

| Quinine hydrochloride concentration | Bitterness |
|---|---|
| 0.004% | 1 (Low) |
| 0.010% | 2 |
| 0.020% | 3 (High) |

### Usage of whey protein hydrolyzate

The whey protein hydrolyzate may be used directly as a protein synthesis promoter, or may be prepared as a powdered drug, granules, a tablet, a capsule, a drinkable preparation, or the like in accordance with a normal method. A whey protein hydrolyzate obtained using an ultrafiltration (UF) membrane or a microfiltration (MF) membrane may be used directly as a protein synthesis promoter, or may be used after drying. The whey protein hydrolyzate may also be prepared as a drug or the like in accordance with a normal method.
The whey protein hydrolyzate that has been prepared as a drug or the like may be added to a nutrient preparation, food or drink (e.g., yogurt, milk-based drink, or wafer), feed, a supplement, or the like.
The content of the whey protein hydrolyzate in the protein synthesis-promoting food, the protein synthesis-promoting drink, the protein synthesis-promoting nutrient composition, or the protein synthesis-promoting feed is not particularly limited, but is preferably determined so that an adult can take the whey protein hydrolyzate in an amount of 10 g/day or more, and preferably 20 g/day or more.
The whey protein hydrolyzate (active ingredient) may be mixed with an appropriate adjuvant, and formed into an arbitrary oral preparation (protein synthesis promoter).

The invention is further described below by way of examples and comparative examples. Note that the invention is not limited to the following examples.

### Example 1

Papain (50 U/g·whey protein) and Proleather (manufactured by Amano Enzyme Inc.) (150 U/g·whey protein) were added to 1 liter of a 10% whey protein aqueous solution. After adjusting the pH of the mixture to 8, the whey protein was hydrolyzed and denatured at 55°C for 6 hours. The reaction solution was heated at 100°C for 15 seconds or more to inactivate the proteases. The reaction solution was then centrifuged, and the supernatant liquid was collected, and dried to obtain a whey protein hydrolyzate (product of Example 1).
The whey protein hydrolyzate (product of Example 1) had a molecular weight distribution that was within a range of 10 kDa or less and had a main peak of 1.3 kDa, an APL of 7.2, and a free amino acid content of 18.9%.
The whey protein hydrolyzate (product of Example 1) had an antigenicity equal to or less than 1/100,000th of that of β-lactoglobulin (measured by inhibition ELISA). The yield (i.e., the ratio (%) of the dry weight of the supernatant liquid to the dry weight of the raw material) was 80.3%, and the bitterness was 2.
The whey protein hydrolyzate (product of Example 1) thus obtained can be used directly as the protein synthesis promoter according to one embodiment of the invention.

### Example 2

Papain (50 U/g·whey protein) and Proleather (150 U/g·whey protein) were added to 1 liter of a 10% whey protein aqueous solution. After adjusting the pH of the mixture to 8, the whey protein was hydrolyzed at 50°C for 3 hours. The mixture was heated to 55°C, and the whey protein was hydrolyzed and denatured at 55°C for 3 hours. Next, the mixture was heated at 100°C for 15 seconds or more to inactivate the proteases. The reaction solution was filtered using a UF membrane having a molecular weight cut-off of 10 kDa (manufactured by STC) and an MF membrane having a molecular weight cut-off of 300 Da (manufactured by STC) to collect a concentrate fraction. The fraction was then dried to obtain a whey protein hydrolyzate (product of Example 2). The whey protein hydrolyzate (product of Example 2) had a molecular weight distribution that was within a range of 10 kDa or less and had a main peak of 500 Da, an APL of 3.0, and a free amino acid content of 15.2%. The whey protein hydrolyzate (product of Example 2) had an antigenicity equal to or less than 1/100,000th of that of β-lactoglobulin (measured by inhibition ELISA). The yield was 65.4%, and the bitterness was 2. The whey protein hydrolyzate (product of Example 2) thus obtained can be used directly as the protein synthesis promoter according to one embodiment of the invention.

### Comparative Example 1

Commercially available branched-chain amino acids (valine, leucine, and isoleucine (all manufactured by Wako Pure Chemical Industries, Ltd.)) were mixed in a ratio of 1:2:1 (i.e., the same ratio as that of Examples 1 and 2) to obtain a product of Comparative Example 1.

### Test Example 1

### Transparency test

A 1% aqueous solution of each whey protein hydrolyzate (Examples 1 and 2 and Comparative Example 1) was prepared, and the absorbance at 650 nm was measured. The results are shown in Table 2.

**TABLE 2**

| Sample | Absorbance (650nm) |
|---|---|
| Product of Example 1 | 0. 008 |
| Product of Example 2 | 0. 004 |
| Product of Comparative Example 1 | 0. 008 |

It was thus confirmed that the whey protein hydrolyzates of Examples 1 and 2 had an absorbance as low as that of the mixture of branched-chain amino acids and had a high transparency.

### Test Example 2

### Protein synthesis promotion test 1

Wistar female rats (7 weeks old, purchased from Japan SLC Inc.) were divided into three groups so that the weight of each group was equal (each group; n=12). The products of Examples 1 and 2 and Comparative Example 1 were respectively dissolved in water so that each solution had the same branched-chain amino acid content. Each solution was orally administered to the rats using a sonde. Immediately after administration, the soleus muscle was removed from three rats of each group under anesthesia, and the ratio of activated (phosphorylated) S6K1 and the ratio of activated (phosphorylated) 4E-BP1 (protein synthesis maker) were determined. When 1, 2, or 3 hours had elapsed after administration, the soleus muscle was removed from three rats of each group under anesthesia, and the ratio of activated (phosphorylated) S6K1 was determined. The results are shown in Table 3. The ratio of activated (phosphorylated) 4E-BP1 was also determined. The results are shown in Table 4.

**TABLE 3**

| Time | Example 1 | Example 2 | Comparative Example 1 |
|---|---|---|---|
| 0 h | 27.5±1.00 | 26.0±1.50 | 27.0±1.00 |
| 1 h | 62.5±2.50 | 60.5±1.00 | 42.5±1.50 |
| 2 h | 46.2±1.50 | 44.5±2.00 | 37.5±2.50 |
| 3 h | 34.5±1.26 | 33.0±1.60 | 32.5±1.25 |
| | | | (%) |

**TABLE 4**

| Time | Example 1 | Example 2 | Comparative Example 1 |
|---|---|---|---|
| 0 h | 2.0±0.50 | 2.0±0.10 | 2.0±0.20 |
| 1 h | 31.5±1.50 | 29.0±1.00 | 17.0±1.00 |
| 2 h | 18.0±1.00 | 16.5±1.50 | 10.0±0.50 |
| 3h | 5.5±1.50 | 6.5-±0.50 | 5.0±1.50 |
| | | | (%) |

### Test Example 3

### Protein synthesis promotion test 2

Wistar female rats (7 weeks old, purchased from Japan SLC Inc.) were divided into three groups so that the weight of each group was equal (n=12). The products of Examples 1 and 2 and Comparative Example 1 were respectively dissolved in water so that each solution had the same branched-chain amino acid content. Each solution was orally administered to the rats using a sonde. Immediately after administration, the liver was removed from three rats of each group under anesthesia, and the ratio of activated (phosphorylated) S6K1 and the ratio of activated (phosphorylated) 4E-BP1 (protein synthesis marker) were determined. When 1, 2, or 3 hours had elapsed after administration, the liver was removed from three rats of each group under anesthesia, and the ratio of activated (phosphorylated) S6K1 was determined. The results are shown in Table 5. The ratio of activated (phosphorylated) 4E-BP1 was also determined. The results are shown in Table 6.

**TABLE 5**

| Time | Example 1 | Example 2 | Comparative Example 1 |
|---|---|---|---|
| 0 h | 22.0±1.00 | 22.0±1.00 | 21.0±1.00 |
| 1 h | 90.5±2.50 | 88.0±2.00 | 43.5±1.50 |
| 2 h | 63.2±1.00 | 64.5±1.00 | 37.5±2.50 |
| 3 h | 35.0±1.00 | 34.0±1.50 | 33.0±1.50 |
| | | | (%) |

**TABLE 6**

| Time | Example 1 | Example 2 | Comparative Example 1 |
|---|---|---|---|
| 0 h | 1.5±0.20 | 1.5±0.20 | 1.5±0.20 |
| 1 h | 46.5±1.50 | 44.0±1.00 | 12-5±1.00 |
| 2h | 26.0±1.00 | 25.0±1.50 | 8.0±0.50 |
| 3h | 4.5±1.00 | 4.0±0.50 | 4.0±1.50 |
| | | | (%) |

As is clear from the results of Test Examples 2 and 3, it was confirmed that the ratio of activated S6K1 and the ratio of activated 4E-BP1 (protein synthesis marker) in the rats that were administered the products of Examples 1 and 2 were higher than those of the rats that were administered the mixture of branched-chain amino acids that are known to be rapidly absorbed. These results suggest that protein synthesis is further promoted by administering the whey protein hydrolyzate as compared with the case of administering the mixture of branched-chain amino acids.

### Example 3

### Production of protein synthesis-promoting tablet

Raw materials were mixed in the ratio shown in Table 7. 1 g of the mixture was formed and tableted using a normal method to produce a protein synthesis-promoting tablet according to one embodiment of the invention.

**TABLE 7**

| | |
|---|---|
| Hydrated crystalline glucose | 73.5 (wt%) |
| Product of Example 1 | 20.0 |
| Mineral mixture | 5.0 |
| Sugar ester | 1.0 |
| Essence | 0.5 |

### Example 4

### Production of protein synthesis-promoting nutrient composition

500 g of the product of Example 2 was dissolved in 4500 g of deionized water. The solution was heated to 50°C, and stirred at 6000 rpm for 30 minutes using a TK-homomixer ("TK ROBO MICS" manufactured by PRIMIX Corporation) to obtain a solution A. 5.0 kg of casein, 5.0 kg of a soybean protein, 1.0 kg of fish oil, 3.0 kg of perilla oil, 18.0 kg of dextrin, 6.0 kg of a mineral mixture, 1.95 kg of a vitamin mixture, 2.0 kg of an emulsifier, 4.0 kg of a stabilizer, and 0.05 kg of essence were added to 5.0 kg of the solution A, and a retort pouch (200 ml) was charged with the mixture. The mixture was then sterilized at 121°C for 20 minutes using a retort sterilizer (class-1 pressure vessel, "RCS-4CRTGN" manufactured by Hisaka Works, Ltd.) to produce 50 kg of a protein synthesis-promoting nutrient composition according to one embodiment of the invention.

### Example 5

### Production of protein synthesis-promoting drink

30 g of a skimmed milk powder was dissolved in 670 g of deionized water, and 10 g of the product of Example 1 was dissolved in the solution. The resulting solution was heated to 50°C, and stirred at 9500 rpm for 30 minutes using an ultra-disperser ("ULTRA-TURRAX T-25" manufactured by IKA Japan). After the addition of 100 g of maltitol, 2 g of an acidifier, 20 g of reduced starch syrup, 2 g of essence, and 166 g of deionized water, a glass bottle (100 ml) was charged with the mixture. After sterilizing the mixture at 90°C for 15 minutes, the bottle was sealed. Ten bottles (100 ml) of a protein synthesis-promoting drink according to one embodiment of the invention were thus obtained.

## Claims

1. A protein synthesis promoter comprising a whey protein hydrolyzate as an active ingredient, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.

2. The protein synthesis promoter according to claim 1, wherein the whey protein hydrolyzate is obtained by performing a hydrolysis step that hydrolyzes and thermally denatures a whey protein at a pH of 6 to 10 and a temperature of 50 to 70°C using a heat-resistant protease, and an inactivation step that inactivates the protease by heating.

3. The protein synthesis promoter according to claim 1, wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, and an inactivation step that inactivates the protease by heating.

4. The protein synthesis promoter according to claim 1, wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, an inactivation step that inactivates the protease by heating, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da.

5. A protein synthesis-promoting food, a protein synthesis-promoting drink, a protein synthesis-promoting nutrient composition, or a protein synthesis-promoting feed comprising the protein synthesis promoter according to any one of claims 1 to 4.

6. A method of producing a protein synthesis promoter comprising a hydrolysis step that hydrolyzes and thermally denatures a whey protein at a pH of 6 to 10 and a temperature of 50 to 70°C using a heat-resistant protease, and an inactivation step that inactivates the protease by heating.

7. The method according to claim 6, further comprising, before the hydrolysis step, a preliminary hydrolysis step that hydrolyzes the whey protein at a pH of 6 to 10 and a temperature of 20 to 55°C using a protease.

8. The method according to claim 6 or 7, further comprising, after the inactivation step, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da.

9. A protein synthesis-promoting method comprising administering a whey protein hydrolyzate in an amount of 10 g/day or more, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.
